(19) Europäisches Patentamt
European Patent Office
Office européen des brevets

(11) **EP 2 593 030 B1**

(12) **EUROPÄISCHE PATENTSCHRIFT**

(45) Veröffentlichungstag und Bekanntmachung des Hinweises auf die Patenterteilung:
**15.07.2020 Patentblatt 2020/29**

(21) Anmeldenummer: **11748255.4**

(22) Anmeldetag: **16.07.2011**

(51) Int Cl.:
*A61B 90/00* (2016.01)        *A61B 34/20* (2016.01)
*G01B 21/04* (2006.01)

(86) Internationale Anmeldenummer:
**PCT/EP2011/003563**

(87) Internationale Veröffentlichungsnummer:
**WO 2012/007179 (19.01.2012 Gazette 2012/03)**

(54) **VERFAHREN ZUR ÜBERPRÜFUNG VON POSITIONSDATEN EINES MEDIZINISCHEN INSTRUMENTES UND ENTSPRECHENDES MEDIZINISCHES INSTRUMENT**

METHOD FOR CHECKING POSITION DATA OF A MEDICAL INSTRUMENT, AND CORRESPONDING MEDICAL INSTRUMENT

PROCÉDÉ DE VÉRIFICATION DES DONNÉES DE POSITION D'UN INSTRUMENT MÉDICAL ET INSTRUMENT MÉDICAL CORRESPONDANT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.07.2010 DE 102010027535**

(43) Veröffentlichungstag der Anmeldung:
**22.05.2013 Patentblatt 2013/21**

(73) Patentinhaber: **Fiagon GmbH**
**10437 Berlin (DE)**

(72) Erfinder:
• **MUCHA, Dirk**
**16548 Glienicke/Nordbahn (DE)**

• **KRUEGER, Timo**
**13469 Berlin (DE)**

(74) Vertreter: **Bobbert & Partner**
**Patentanwälte PartmbB**
**Postfach 1252**
**85422 Erding (DE)**

(56) Entgegenhaltungen:
**EP-A1- 1 632 168          EP-A1- 2 186 474**
**WO-A1-2005/082246     WO-A1-2010/133320**
**DE-A1-102008 057 744**

**Beschreibung**

Die vorliegende Erfindung betrifft ein Verfahren zur Überprüfung von Lage- oder Positionsdaten eines medizinischen Instrumentes gemäß dem Oberbegriff des Anspruchs 1 sowie eine Vorrichtung gemäß dem Oberbegriff des Anspruchs 7. Sie betrifft ferner ein digitales Speichermedium gemäß Anspruch 15.

Beim Arbeiten mit einem Instrument in einem Bereich, der nicht direkt einsehbar ist, kann es von Bedeutung sein, die aktuelle Lage des Instrumentes zu erfassen und in geeigneter Weise darzustellen. Bekannt ist, ein Instrument mit elektromagnetischen Lagesensoren, sogenannten Spulenelementen, zu versehen. Ein Feldgenerator, der in räumlicher Nähe des Instrumentes angeordnet ist, erzeugt ein elektromagnetisches Feld, das elektrische Spannungen in den Spulenelementen des Instrumentes nach dem Gesetz der elektromagnetischen Induktion induziert. Die Höhe der induzierten Spannungen bzw. der elektrischen Ströme in den Spulenelementen kann je nach räumlicher Lage und Positionierung der Sensoren an dem Instrument unterschiedlich sein. Die elektromagnetischen Lagesensoren können ebenso sogenannte Saturationskernmagnetometer sein, die ein Signal liefern, das proportional zur lokalen auftretenden Feldstärke des erzeugten elektromagnetischen Feldes ist.

Eine Steuerungseinheit, die mit dem Feldgenerator und den Lagesensoren verbunden ist, kann anschließend aus den verschiedenen Messdaten des Feldgenerators und der Lagesensoren die Position der Sensoren und damit des Instrumentes berechnen. Diese können beispielsweise auf einem Monitor dargestellt werden.

Bekannt sind auch Verfahren zur Lagebestimmung von Instrumenten, die auf optischen Messprinzipien von geeigneten Sensoren auf dem Instrument beruhen.

Weiterhin sind Fehlererkennungsverfahren für Instrumente bekannt, die starr sind und sich während der Anwendung der Instrumente nicht verformen. Diese Fehlererkennungsverfahren basieren auf dem Prinzip, dass zwei verschiedene Sensoren auf dem Instrument zu einem Zeitpunkt einen bestimmten Bereich des Instrumentes unterschiedlich referenzieren und somit unterschiedliche Positionsdaten bestimmen. Dadurch wird auf einen Fehler bei der Messung zumindest von einem Sensor geschlossen. Werden derartige Verfahren bei Instrumenten eingesetzt, die sich während der Anwendung verformen, können die Lagedaten des verformten Instruments nicht eindeutig von möglichen fehlerhaften Lagedaten unterschieden werden. Insbesondere werden auch durch die Verformung des Instruments Lagedaten als fehlerhaft eingestuft, obwohl eine Störung nicht vorliegt.

Aus der DE 10 2009 021 705 A1 ist ein Verfahren zum Erzeugen von Positionsdaten und/oder zum Erkennen von Deformationen eines Instrumentes bekannt.

EP 1632168 A1 bezieht sich auf ein System, das die Form eines Einführungsabschnitts eines Endoskops unter Verwendung von Magnetfelderzeugungselementen und Magnetfelddetektionselementen erfasst und die erfasste Form anzeigt. Es wird berücksichtigt, ob die Krümmung der Verformung des Endoskops zu hoch ist, was zu einem Fehler in der Positionsmessung führen kann.

Aufgabe der vorliegenden Erfindung ist es, ein Verfahren vorzuschlagen, das die Erkennung fehlerhaft ermittelter Lage- oder Positionsdaten eines Instruments oder deren Überprüfung erlaubt.

Diese Aufgabe wird gelöst durch die Merkmalskombination des Anspruchs 1.

Bei diesem Verfahren weist das medizinische Instrument wenigstens zwei verschiedene Abschnitte, einen ersten und einen zweiten Abschnitt, auf. Die Form und/oder Funktion der beiden Abschnitte kann jeweils gleich oder unterschiedlich sein. Der erste Abschnitt weist wenigstens einen ersten Sensor auf, der zweite Abschnitt weist ebenfalls wenigstens einen Sensor auf, im Folgenden als zweiter Sensor bezeichnet. Das Verfahren umfasst ein messtechnisches Bestimmen der Lage bzw. der Position im Raum, oder deren Veränderung, des ersten Sensors und des zweiten Sensors.

Vorteilhafte Weiterentwicklungen der erfindungsgemäßen Aufgabe sind jeweils Gegenstand der Unteransprüche.

In einigen erfindungsgemäßen Ausführungsformen wird basierend auf der Ermittlung anhand des Kriteriums eine Unterscheidung getroffen, ob der Unterschied in der Ausprägung des veränderlichen Merkmals zwischen der ersten Ausprägung zum ersten Zeitpunkt ($t_1$) und der zweiten Ausprägung zum zweiten Zeitpunkt ($t_2$) auf einer Deformation oder Biegung oder Verbiegung des Instruments oder auf einer anderen Beeinflussung des gemessenen räumlichen Bezugs wie beispielsweise einem Artefakt oder einer Störung beruht.

Der Begriff "Lage", wie er hierin verwendet wird, beschreibt in bestimmten Ausführungsformen der vorliegenden Erfindung die Lage eines Sensors als Position und Ausrichtung eines dreidimensionalen geometrischen Körpers im Raum.

Der Begriff Lage kann synonym zu dem Begriff Lagedaten verwendet werden. Die Lagedaten beschreiben quantitativ die Lage in einem Bezugssystem.

Der Begriff "Position", wie er hierin verwendet wird, beschreibt in manchen Ausführungsformen der vorliegenden Erfindung den Ort eines dreidimensionalen geometrischen Körpers im Raum.

Der Begriff Position kann synonym zu dem Begriff Positionsdaten verwendet werden. Die Positionsdaten beschreiben quantitativ die Position in einem Bezugssystem.

In einem Schritt des erfindungsgemäßen Verfahrens wird eine Ausprägung eines veränderlichen Merkmals des räumlichen Bezugs zwischen der Lage des ersten Sensors und der Lage des zweiten Sensors wenigstens zu jeweils drei Zeitpunkten, einem ersten Zeitpunkt, einem zweiten Zeitpunkt und einem dritten Zeitpunkt bestimmt.

In einem weiteren Schritt wird anhand eines, insbesondere vorbestimmten, Kriteriums ermittelt, ob ein Unterschied zwischen einer ersten Ausprägung des veränderlichen Merkmals zum ersten Zeitpunkt und einer zweiten Ausprägung zum zweiten Zeitpunkt des veränderlichen Merkmals zum dritten Zeitpunkt noch besteht.

Durch Kenntnis des erfindungsgemäß ermittelbaren Unterschieds der Ausprägung des veränderlichen Merkmals einerseits zwischen der ersten Ausprägung zum ersten Zeitpunkt und der zweiten Ausprägung zum zweiten Zeitpunkt und andererseits dessen Fortbestehen (der jeweiligen Ausprägungen) zum dritten Zeitpunkt anhand eines Kriteriums kann in bestimmten erfindungsgemäßen Ausführungsformen eine Fehlererkennung von Lagedaten und/oder das Erkennen einer vorliegenden Deformation des Instruments vorteilhaft möglich sein.

Die Bestimmung der Lage oder der Lagen des ersten Sensors und des zweiten Sensors zueinander kann rechnerisch erfolgen.

Der Begriff "zueinander", wie er hierin verwendet wird, beschreibt die Lage des ersten Sensors in Bezug auf den zweiten Sensor. Ebenso kann die Lage des zweiten Sensors in Bezug auf den ersten Sensor beschrieben werden.

Die Lage eines Sensors kann durch dessen Koordinaten in einem Koordinatensystem dargestellt werden.

Die mathematische Lagebeschreibung eines Sensors, der im Sinne der Erfindung auch als Lokalisator oder als Lagesensor bezeichnet werden kann, kann unter Verwendung eines Koordinatensystems mittels Matrixdarstellung erfolgen.

Für die rechnerische oder mathematische Beschreibung der Lage eines ersten Sensors in Bezug auf die Lage eines zweiten Sensors können Matrizen angewendet werden. Die Matrix des ersten Sensors (synonym zu: die Matrix der Position des ersten Sensors, dargestellt oder beschrieben durch dessen Matrix als Positionsbeschreibung im Raum) kann auf die Matrix des zweiten Sensors transformiert werden. Die Lagebeschreibung des ersten Sensors in Bezug auf den zweiten Sensor (oder umgekehrt) wird dann durch eine Transformationsmatrix beschrieben.

Durch die Transformationsmatrix können die Koordinaten des ersten Sensors auf den zweiten Sensor transformiert werden. Anders ausgedrückt wird eine Koordinatentransformation der Koordinaten des ersten Sensors auf die Koordinaten des zweiten Sensors durchgeführt.

Ein veränderliches Merkmal des räumlichen Bezugs kann eine Koordinatentransformation oder deren Ergebnis sein.

Ein veränderliches Merkmal kann ein zeitlich veränderliches Merkmal sein.

Ein zeitlich veränderliches Merkmal kann zu einem ersten Zeitpunkt $t_1$, zu einem zweiten Zeitpunkt $t_2$, zu einem dritten Zeitpunkt $t_3$ und ferner ergänzend zu weiteren Zeitpunkten $t_i$ (ti für i=4, 5, 6, 7,...) betrachtet werden.

In einigen erfindungsgemäßen Ausführungsformen liegen alle betrachteten Zeitpunkte $t_i$ (ti für i= 1,2,3,4,5,6,7, ...), in anderen erfindungsgemäßen Ausführungsformen nur die Zeitpunkte $t_1$, $t_2$ und $t_3$, jeweils nach Beginn einer Behandlung des Patienten mittels des Instruments.

Bei einer Deformation des Instrumentes kann die Ausprägung des veränderlichen Merkmals (welche dann als bleibende Veränderung oder Ausprägung bezeichnet werden kann) des räumlichen Bezugs bei einem dritten Zeitpunkt ($t_3$) als neue Ausgangsausprägung gesetzt werden. Das deformierte Instrument zum Zeitpunkt $t_3$ kann dann als Ausgangsausprägung für weitere Zeitpunkte $t_i$ ($t_i$ für i=4, 5, 6, 7, ...) betrachtet werden.

Die Koordinatentransformation kann vom ersten Sensor auf den zweiten Sensor mathematisch beschrieben werden, ebenso wie die Koordinatentransformation vom zweiten Sensor auf den ersten Sensor. Gleichfalls ist die Koordinatentransformation vom zweiten Sensor auf den ersten Abschnitt des Instrumentes möglich, ebenso wie die Koordinatentransformation vom ersten Sensor auf den zweiten Abschnitt. Weiterhin ist die Koordinatentransformation vom ersten Sensor auf den ersten Abschnitt möglich, wie auch die Koordinatentransformation vom zweiten Sensor auf den zweiten Abschnitt.

Der "erste Abschnitt" oder der "zweite Abschnitt", oder ein Punkt des ersten oder zweiten Abschnitts, kann die Spitze des Instruments oder ein medizinisch wirksamer Abschnitt hiervon, z. B. eine Schneidefläche, sein. Der erste oder zweite Abschnitt kann alternativ jeder andere Abschnitt sein oder einen solchen umfassen.

Jeder der vorgesehenen Sensoren kann auf der Oberfläche des Instrumentes oder innerhalb des Instrumentes bzw. der Abschnitte angeordnet sein. Beispielsweise kann es vorteilhaft sein, wenigstens einen Sensor im Instrument zu kapseln, wenn er nicht mit der Umgebung in Berührung kommen soll. Dies kann entweder zum Schutz des Sensors vor dem Umgebungsmedium dienen, aber auch umgekehrt dem Schutz des Umgebungsmediums oder -gewebes vor dem Sensor. Mögliche Anwendungsfälle sind Messungen in toxischen und/oder aggressiven Substanzen, die den Sensor zerstören könnten oder Anwendungen in der Umgebung von humanem oder tierischem Gewebe, das durch Sensormaterialien beeinflusst werden kann.

Der Sensor kann eine oder mehrere Spulen beinhalten oder hieraus jeweils bestehen.

Die Spule oder die Spulen haben in bestimmten Ausführungsformen ein durchgängiges Inneres. Dieses durchgängige Innere kann eine beliebige Querschnittsform aufweisen; beispielsweise kann es rund, oval, rechteckig oder vieleckig sein. Die Durchgängigkeit des Inneren kann in einer Längsrichtung der Spule bestehen. Die Durchgängigkeit des Inneren kann in einem Gebrauchszustand des Instruments, mit welchem die Spule verbunden ist, in einer Längsrichtung des Instruments oder eines Abschnitts, welcher die Spule aufweist, bestehen.

Unter einer Durchgängigkeit ist in bestimmten Ausführungsformen die Möglichkeit zu verstehen, durch das Innere der Spule hindurch z. B. ein weiteres Instrument, d. h. ein anderes als jenes, mit welchem die Spule in ihrem Gebrauchszustand im Sinne der vorliegenden Erfindung fest verbunden ist, geführt oder geschoben werden kann. Eine Durchgängigkeit kann in manchen Ausführungsformen als die Möglichkeit verstanden werden, durch die Spule hindurch zu agieren.

Die Spule oder die Spulen kann oder können um einen Abschnitt des Instruments, mit welchem sie gemeinsam oder fest verbunden im Sinne der vorliegenden Erfindung verwendet wird oder werden, herum angeordnet sein.

Eine jede Spule kann alternativ innerhalb des Abschnitts des Instruments, mit welchem sie gemeinsam oder fest verbunden im Sinne der vorliegenden Erfindung verwendet wird, angeordnet sein.

Eine Spule (oder mehrere oder alle Spulen) kann aus Windungen aufgebaut sein.

Der Abschnitt des Instruments, welcher Sensoren oder Spulen trägt, kann einen wenigstens in Teilen des Abschnitts rohrförmigen (hohlen) Querschnitt aufweisen. Der Querschnitt kann auch jede andere beliebige Form aufweisen, insbesondere solide oder nicht-hohl sein.

Die Sensoren können auf unterschiedlichen physikalischen Messprinzipien beruhend messen oder arbeiten.

In bestimmten erfindungsgemäßen Ausführungsformen werden elektromagnetische Sensoren verwendet. Dies hat den Vorteil, dass beispielsweise gegenüber optischen Sensoren die Sensoren innerhalb verschiedener Medien, Gehäuse oder Verkapselungen verwendet werden können, ohne dass eine direkte Sichtverbindung notwendig ist.

Weiterhin können sich die Sensoren, die sich auf dem ersten Abschnitt befinden, von den Sensoren des zweiten Abschnitts unterscheiden. Ein erster Sensor kann sich in den Abmaßen, in der Sensitivität, der Messgenauigkeit, den Gehäusematerialien des Sensors und anderen Merkmalen von einem und/oder weiteren Sensoren unterscheiden. Auch die - insbesondere physikalischen - Messprinzipien, auf welchen basierend die Sensoren arbeiten, können verschieden sein. Ein erster Sensor kann beispielsweise auf einem elektromagnetischen Messprinzip beruhen, ein weiterer Sensor beispielsweise auf einem optischen Messprinzip.

Die messtechnische Bestimmung der Lage der Sensoren hängt von den verwendeten Messprinzipien der Sensoren ab und wird hier nicht weiter vertieft, da sie dem Fachmann hinreichend bekannt ist.

Die Koordinatentransformation kann zu verschiedenen Zeitpunkten bestimmt oder berechnet werden. Wenn beispielsweise ein Instrument mit einem oder mehreren Abschnitten ein oder mehrere Sensoren aufweist, können die jeweiligen Koordinatentransformationen zu einem ersten Zeitpunkt $t_1$ und zu weiteren Zeitpunkten $t_i$ (i=2, 3, 4,...) bestimmt werden.

Beispiele für derartige Koordinatentransformationen, die im folgenden durch homogene Transformationsmatrizen (T) mathematisch beschrieben werden, können sein:

$^{S1}T_{S2}$ $(t_1)$ — Koordinatentransformation des ersten Sensors auf den zweiten Sensor (zum ersten Zeitpunkt $t_1$; allgemein: die Lage des ersten Sensors wird in Bezug auf den zweiten Sensor zum ersten Zeitpunkt $t_1$ bestimmt bzw. berechnet; oder: die Lage des ersten Sensors und die Lage des zweiten Abschnitts zueinander wird bestimmt bzw. rechnerisch ermittelt bzw. berechnet;

$^{S2}T_{A1}$ $(t_1)$ — Koordinatentransformation des zweiten Sensors auf den ersten Abschnitt zum ersten Zeitpunkt $t_1$;

$^{S1}T_{A1}$ $(t_1)$ — Koordinatentransformation des ersten Sensors auf den ersten Abschnitt zum ersten Zeitpunkt $t_1$

Die Koordinatentransformation vom ersten Sensor auf den ersten Abschnitt ($^{S1}T_{A1}$) kann bei einem starren ersten Abschnitt des Instruments als annähernd zeitlich konstant angesehen werden. Entsprechendes gilt für den zweiten Abschnitt und den zweiten Sensor.

Der Begriff "starrer erster Abschnitt", wie er hierin verwendet wird, beschreibt einen nicht verformbaren oder bei gewöhnlichem Einsatz des Instruments vernachlässigbar gering verformbaren oder unterhalb der Messgenauigkeit verformbaren Abschnitt.

Der Begriff "annähernd zeitlich konstant", wie er hierin verwendet wird, bedeutet zeitlich konstant im Rahmen der Meßgenauigkeit zu zwei aufeinander folgenden Zeitpunkten (z. B. liegen die ersten und zweiten Zeitpunkte $t_1$ und $t_2$ eine Sekunde oder zwei Sekunden oder fünf Sekunden auseinander):

$$^{S1}T_{A2}\ (t_1)\ \cong\ ^{S1}T_{A2}\ (t_2).$$

Erfindungsgemäß wird anhand eines Kriteriums ermittelt, ob ein Unterschied des veränderlichen Merkmals zwischen einer ersten Ausprägung zum ersten Zeitpunkt $t_1$ und einer zweiten Ausprägung zum zweiten Zeitpunkt $t_2$ auch zu einem dritten Zeitpunkt ($t_3$) noch besteht.

Ein veränderliches Merkmal des räumlichen Bezugs kann als eine Koordinatentransformation zwischen dem ersten und zweiten Sensor bestehen. Die Ausprägung kann durch eine erste Position (zum ersten Zeitpunkt $t_1$) oder eine zweite Position (zum zweiten Zeitpunkt $t_2$) bestimmt sein.

Weitere Beispiele für das veränderliche Merkmal umfassen eine Geschwindigkeit oder eine Beschleunigung der Koordinatentransformation zu einem bestimmten Zeitpunkt oder in einem bestimmten Zeitraum.

Ein weiteres Beispiel für das veränderliche Merkmal kann die Lage des ersten Sensors und des zweiten Sensors im Raum sein (Lagemesswerte), die als Koordinaten im Koordinatensystem des Lagemesssystems bestimmt werden. Diese Lagen können ebenfalls als Koordinatentransformation dargestellt werden.

Als Basis für die Koordinatentransformation (als räumlicher Bezug) können die Freiheitsgrade eines Koordinatensystems angesehen werden. Beispielsweise hat die Koordinatentransformation $^{S1}T_{S2}$ zum ersten Zeitpunkt $t_1$ drei translatorische und drei rotatorische Freiheitsgrade in den Achsrichtungen des jeweiligen Koordinatensystems. Bei einem x,y,z-Koordinatensystem sind dies die translatorischen Freiheitsgrade in x-, y- und z-Richtung und die rotatorischen Freiheitsgrade um die x-, y- und z-Achse.

Kriterien für die Veränderung des veränderlichen Merkmals können beispielsweise sein:

- Die Änderung eines Freiheitsgrads der Koordinatentransformation zwischen dem ersten und zweiten Sensor
- Die Standardabweichungen eines Freiheitsgrads der Koordinatentransformation zwischen dem ersten und zweiten Sensor über n Messwerte der Sensoren
- Die lineare Zuordnung eines Freiheitsgrads des Lagemesswerts des ersten Sensors zu dem entsprechenden Freiheitsgrad des Lagemesswerts des zweiten Sensors
- Auftretende mittlere Geschwindigkeit eines Freiheitsgrads der Koordinatentransformation zwischen dem ersten und dem zweiten Sensor über n Messwerte der Sensoren
- Auftretende Geschwindigkeiten eines Freiheitsgrads der Koordinatentransformation zwischen dem ersten und dem zweiten Sensor über n Messwerte der Sensoren
- Auftretende Beschleunigung eines Freiheitsgrads der Koordinatentransformation zwischen dem ersten und zweiten Sensor über n Messwerte der Sensoren

Diese Kriterien können einzeln oder in beliebigen Kombinationen gemeinsam angewendet werden. In einer erfindungsgemäßen Ausgestaltung können z. B. auch die Änderungen aller sechs Freiheitsgrade der Koordinatentransformation zwischen dem ersten Sensor und dem zweiten Sensor gemeinsam als Kriterium für eine Veränderung angewendet werden.

Der Begriff "lineare Zuordnung", wie er hierin verwendet wird, bedeutet eine lineare Zuordnung (auch als lineare Zuordnung oder lineare Funktion bezeichnet) der Lagemesswerte vom ersten Sensor und vom zweiten Sensor. Die entsprechenden Freiheitsgrade der Messdaten vom ersten Sensor und vom zweiten Sensor haben in bestimmten erfindungsgemäßen Ausführungsformen über wenigstens drei aufeinanderfolgenden Messwerte eine lineare Zuordnung.

Dabei weisen die Messdaten in manchen erfindungsgemäßen Ausführungen nur dann eine lineare Zuordnung auf, wenn bei allen Freiheitsgraden der Lagemesswerte der lineare Zusammenhang über wenigstens drei aufeinanderfolgende Messwerte nachgewiesen wird. Wenn beispielsweise alle translatorischen Freiheitsgrade lineare Zuordnungen aufweisen, die rotatorischen Freiheitsgrade (Drehbewegungen um die Koordinatenachsen) jedoch ausreichend unterschiedlich sind (beispielsweise einander entgegengesetzte Rotationsbewegungen), so weist die Koordinatentransformation an den drei aufeinanderfolgenden Zeitpunkten insgesamt keine lineare Zuordnung auf.

Der Grad der Linearität kann anhand eines Korrelationskoeffizienten gemessen werden. Bei einem Korrelationskoeffizienten von Eins (1) oder nahe Eins (1) besteht eine hohe Linearität; bei Null (0) oder nahe Null (0) besteht keine oder nur eine geringe Linearität.

Erfindungsgemäß können beim Ermitteln der Unterschiede der Koordinatentransformationen zwischen den einzelnen Zeitpunkten mittels der Kriterien in bestimmten Ausführungsformen vorbestimmte Toleranzbereiche beachtet werden.

Diese Toleranzbereiche können für die lineare Zuordnung bestimmt werden (Toleranzbereich des Korrelationskoeffizienten "nahe Eins" zur Entscheidung, ob eine lineare Zuordnung vorhanden ist oder nicht), für die mittlere Geschwindigkeit (Toleranzbereich der mittleren Geschwindigkeit "nahe Null" zur Entscheidung "ja" oder "nein"), für die absoluten, auftretenden Geschwindigkeiten (Toleranzbereich der Geschwindigkeiten "nahe Null" zur Entscheidung "ja" oder "nein") und für die auftretenden Beschleunigungen (Toleranzbereich der Beschleunigungen "nahe Null" zur Entscheidung "ja" oder "nein").

Weiterhin können erfindungsgemäß beim Ermitteln der Unterschiede der Koordinatentransformationen zwischen den einzelnen Zeitpunkten mittels der Kriterien Grenzwerte für die Standardabweichungen der Koordinatentransformationen beachtet werden.

In bestimmten Ausführungsformen der Erfindung werden eine Deformation des Instrumentes und/oder Störeinflüsse bei dem messtechnischen Bestimmen der Sensoren unter Anwendung des Kriteriums und/oder des vorbestimmten Toleranzbereichs und/oder des vorbestimmten Grenzwerts bestimmt oder voneinander unterschieden.

Das Verfahren kann erfindungsgemäß folgende Schritte enthalten:

Zu jedem Zeitpunkt wird die Transformation zwischen dem ersten und dem zweiten Sensor erfasst. Diese ist bei keiner Störung und keiner Deformation konstant gegenüber einem Sollwert, bzw. Variationen liegen unterhalb eines Grenzwerts (Normalzustand). Der Sollwert wird aus den Sensorwerten zu einem ersten Zeitpunkt $t_1$ als Transformation zwischen den Sensoren bestimmt und hinterlegt, oder liegt bereits gespeichert vor.

Tritt eine Änderung der gemessenen Transformation zwischen den Sensoren, zum Sollwert auf, bedeutet dies, dass entweder eine Deformation vorliegt oder eine Störung vorliegt. Bei der Deformation ist die physische Lage der Sensoren zueinander permanent (bis zur nächsten Deformation) verändert, so dass der Sollwert der Transformation nicht mehr zutrifft. Die Abweichung von dem Sollwert ist (nach der Deformation) konstant. Dies wird durch die beschriebenen Kriterien erkannt.

Bei einer Störung entspricht die physische Lage der Sensoren zueinander noch dem Sollwert, jedoch wird durch die Störung eine fehlerhafte Lage mindestens eines Sensors erfasst. Dies resultiert in einer nur für die Dauer der Störung auftretenden Abweichung der Transformationsmatrix zu dem Sollwert. Des Weiteren sind die Abweichungen, die durch eine Störung hervorgerufen werden (auch während der Störung) nicht konstant. Dies wird durch die oben beschriebenen Kriterien erkannt.

Bei jedem Auftreten einer Abweichung vom Sollwert wird entschieden, ob eine Deformation oder eine Störung vorliegt. Dazu dienen die oben beschriebenen Kriterien.

Liegt eine Deformation vor, wird die Abweichung der gemessenen Transformation zwischen den Sensoren von dem Sollwert der Transformationsmatrix berechnet. Das Ergebnis wird zur Berechnung einer neuen Transformationsmatrix zwischen dem zweiten Sensor und der Instrumentenspitze verwendet. Die gemessene Transformation zwischen den Sensoren wird als neuer Sollwert gesetzt und kann hinterlegt werden.

Dadurch ist bis zum erneuten Auftreten einer Störung oder Deformation wieder der Normalzustand hergestellt. Das Verfahren läuft entsprechend oben stehender Beschreibung in einer Schleife weiter.

Liegt eine Störung vor, werden die Messwerte bis zum Abklingen der Störung beispielsweise durch einen geeigneten Filter bearbeitet. Das Abklingen der Störung kann durch die bereits beschriebene Beobachtung der Abweichung der gemessenen Transformation zwischen den Sensoren von dem Sollwert der Transformationsmatrix zu jedem Zeitpunkt erfasst werden. Nach Beseitigung oder Abklingen der Störung läuft das Verfahren entsprechend oben stehender Beschreibung in einer Schleife weiter.

Die Kriterien können erfindungsgemäß beispielsweise folgendermaßen verwendet werden:
Eine Deformation des Instruments kann beispielsweise durch eine lineare Zuordnung zwischen den einzelnen Freiheitsgraden den Messdaten vom ersten Sensor und vom zweiten Sensor über wenigstens drei aufeinanderfolgende Messwerte nach der Deformation bestimmt werden.

Beispielhaft kann eine Biegung oder Verbiegung des Instruments zum Zeitpunkt $t_2$ betrachtet werden, die zum Zeitpunkt $t_1$ noch nicht vorgelegen hat. Die plastische Deformation liegt dann zum Zeitpunkt $t_2$ vor (und bleibt über nachfolgende Zeitpunkte erhalten). Zur Unterscheidung der Deformation von Störeinflüssen können die Lagemessdaten des ersten und des zweiten Sensors betrachtet werden. Die Lagemessdaten können je durch eine Transformationsmatrix beschrieben werden. Diese Transformationsmatrizen weisen zu allen Zeitpunkten jeweils 6 Freiheitsgrade (3 translatorische und 3 rotatorische Freiheitsgrade) auf, die die Koordinatentransformation vom ersten Sensor bzw. vom zweiten Sensor zum Koordinatensystem des Messsystems (oder umgekehrt) beschreiben können ($T_a$ bzw. $T_b$). Wenn z. B. zwischen allen 6 entsprechenden Freiheitsgraden der Koordinatentransformationen $T_a$ und $T_b$ über z. B. drei aufeinanderfolgende Messwerte nach der Deformation $T_a(t_2)$ und $T_b(t_2)$, $T_a(t_3)$ und $T_b(t_3)$ und $T_a(t_4)$ und $T_b(t_4)$ ein linearer Zusammenhang besteht, kann eine Deformation des Instrumentes zwischen den Zeitpunkten $t_1$ und $t_2$ bestimmt werden. Dasselbe gilt, wenn keine Rückkehr aus einer sich von der Ausgangslage unterscheidenden Lage zur Ausgangslage beobachtet werden kann.

Eine fehlerhafte Positionsermittlung, bedingt durch Störeinflüsse, kann dagegen bestimmt oder erkannt werden, wenn die lineare Zuordnung zwischen wenigstens einem entsprechenden Freiheitsgrad der Koordinatentransformationen $T_a$ und $T_b$ über wenigstens drei aufeinanderfolgende Zeitpunkte, z. B. $t_2$ bis $t_4$ nicht vorliegt oder eine Rückkehr in die Ausgangslage erkannt wird.

In manchen erfindungsgemäßen Ausführungen wird eine Deformation des Instruments durch die mittlere Geschwindigkeit der einzelnen Freiheitsgrade der Koordinatentransformationen an wenigstens drei aufeinanderfolgenden Zeitpunkten bestimmt.

Eine Deformation des Instruments kann dann bestimmt werden, wenn die mittlere Geschwindigkeit der einzelnen Freiheitsgrade der Koordinatentransformationen von wenigstens zwei aufeinanderfolgenden Messwerten nach der Deformation nahe Null (als Beispiel eines Kriteriums) ist. Eine Geschwindigkeit nahe Null bedeutet, dass die gemessenen Sensorsignale an den drei aufeinanderfolgenden Zeitpunkten nahe aneinander liegen und daher auf eine Deformation des Instruments, nicht aber auf einen Fehler bei der Positionsermittlung geschlossen werden kann.

Eine Fehlererkennung kann dagegen angenommen werden, wenn die Geschwindigkeit nicht nahe Null ist. Eine Geschwindigkeit nicht nahe Null kann bedeuten, dass die gemessenen Sensorpositionen sich relativ zueinander bewegen. Dies entspricht nicht der physikalischen Gegebenheit, bei der die Sensoren (nach der Deformation) wieder "starr" zueinander angeordnet sein sollten. Daher können die gemessenen Sensorsignale als Störsignale interpretiert werden. Diese werden beispielsweise verworfen.

In der gleichen Weise werden gemessene Sensorsignale, welche die absoluten Geschwindigkeiten und auftretende Beschleunigungen aufzeigen, zum Erkennen von Deformationen oder Fehlern in manchen erfindungsgemäßen Ausführungsformen verwendet.

Die vorliegende Erfindung betrifft insbesondere die Überprüfung einer Positionsbestimmung von Instrumenten oder Abschnitten hiervon, die sich während ihrer Anwendung verformen, sei dies aufgrund einer beabsichtigten oder einer nicht beabsichtigen Verformung oder Deformation des Instrumentes. Eine Verformung wird hier in dem Sinn beschrieben, dass der zweite Abschnitt seine Lage in Bezug auf den ersten Abschnitt verändert. Je nach Geometrie und Materialbeschaffenheit des Instruments, nach physikalischen Umgebungsparametern wie beispielsweise Temperatur und Druck sowie nach den voraussichtlich angreifenden Kräften und Momenten an dem Instrument kann es möglich sein, die voraussichtliche Verformung mathematisch zu beschreiben oder mit ausreichender Genauigkeit zu approximieren.

In einer weiter bevorzugten Ausführungsform der Erfindung ist eine Spitze des Instruments der Bezugspunkt zum Erzeugen der Positionsdaten des zweiten Abschnitts des Instruments.

In einer wiederum weiter bevorzugten Ausführungsform der Erfindung werden die mittels der Sensoren gemessenen Positionsdaten oder deren nachfolgende Weiterverarbeitung in einer Steuereinheit oder in einem Computer auf einer geeigneten Ausgabeeinrichtung ausgegeben. Eine derartige Ausgabeeinrichtung kann beispielsweise ein Monitor sein.

Hierbei kann es sinnvoll sein, Messdaten, die zur Fehlererkennung bestimmt wurden bzw. als Störsignale gemessen wurden und/oder die außerhalb eines Grenzwertbereiches liegen, der zuvor festgelegt worden ist, nicht auszugeben, nicht in weiteren Betrachtungen zu verwenden, usw. Dies kann beispielsweise durch einen Schwellenwertfilter in einer entsprechenden Recheneinheit realisiert werden.

In bestimmten erfindungsgemäßen Ausführungsformen des Verfahrens wird wenigstens ein weiterer Sensor auf jeweils einem weiteren Abschnitt verwendet, also z. B. ein dritter, vierter, usw. Sensor auf jeweils einem dritten, vierten, usw. Abschnitt. Bei den weiteren Sensoren wird wie oben beschrieben der räumliche Bezug zwischen Lage oder Position des oder jedes einzelnen weiteren Sensors einerseits und dem ersten Sensor und/oder wenigstens eines Punktes auf dem ersten Abschnitt andererseits bestimmt.

Von der vorliegenden Erfindung umfasst ist auch das Vorsehen nur eines Schwellenwertes.

Die Nichtausgabe von Messwerten unter- oder oberhalb eines Grenzwertes kann dazu geeignet sein, die Positionsbestimmung von Instrumenten in ihrer Genauigkeit zu verbessern und die Zuverlässigkeit bezüglich der Ausgabe der tatsächlichen Position des Instrumentes vorteilhaft zu erhöhen. Die - vor allem unbemerkte - Beeinflussung durch Gegenstände im Umfeld des Instrumentes, genauer im Messbereich der Sensoren auf dem Instrument, wird durch das beschriebene Verfahren vorteilhaft verringert. Bei den beschriebenen elektromagnetischen Sensoren (Spulenelementen) gilt dies beispielsweise für die Beeinflussung von Gegenständen, die ferromagnetische Eigenschaften aufweisen. Die Feldstörung durch diese Gegenstände oder ihr Einfluss auf eine Positionsermittlung wird durch das genannte Verfahren, besonders für nicht-starre Instrumente, die für gewollte Deformationen geeignet sind, und für starre, jedoch deformierbare Instrumente mit Lagesensoren verringert.

In einer wiederum weiter bevorzugten Ausführungsform wird das erfindungsgemäße Verfahren bei den im Folgenden beschriebenen medizinischen Instrumenten angewendet. Beispielsweise in der Mund-, Kiefer-, Gesichtschirurgie als auch im Hals-Nasen-Ohren Bereich kann es während operativen Eingriffen sehr wichtig sein, die genaue Position von Operationsinstrumenten zu kennen, um nicht Gefäße, Nerven oder andere sensible Bereiche zu verletzen. Gleichzeitig werden bei derartigen Operationen eine Vielzahl von Instrumenten, Überwachungs- und Diagnosegeräten und anderen Hilfsmitteln für die Operation eingesetzt. Oftmals besteht eine Vielzahl von diesen Instrumenten und Geräten aus metallischen Werkstoffen, beispielsweise um eine Sichtbarkeit auf Computertomographien zu erreichen. Ein weiterer Grund zum heute verbreiteten Einsatz von metallischen Werkstoffen bei Operationsinstrumenten ist die gute Sterilisierbarkeit, die gerade im medizinischen Bereich von großer Bedeutung ist. Andererseits finden neue operative Operationstechniken wie beispielsweise die minimal-invasive Chirurgie immer größere Anwendungsfelder, die oft ein genaues Navigieren und Führen von Instrumenten in nicht direkt einsehbaren Gebieten erfordern. Auch in den zuvor genannten Einsatzbereichen bietet das erfindungsgemäße Verfahren große Vorteile gegenüber dem aus dem Stand der Technik Bekannten.

Das verwendete Instrument kann beispielsweise eine Ultraschallsonde (Rektal-, Vaginalsonde, intravaskuläre Sonde), ein Katheter, ein Sauger, ein Endoskop oder ein Zeigeinstrument sein. Oft kann es vorteilhaft sein, wenn zumindest ein Abschnitt ein geometrisch definiertes Ende aufweist, um beispielsweise als Zeigeinstrument geeignet anwendbar zu sein.

Mit Hilfe des erfindungsgemäßen Verfahrens ist es vorteilhaft möglich, die Positionsbestimmung von Instrumenten, die sich während der Anwendung verformen, zu überprüfen, und/oder erkannte Artefakte mitzuteilen oder in einer Positionsbestimmung zu berücksichtigen.

Die erfindungsgemäße Aufgabe wird auch gelöst durch die Vorrichtung gemäß dem Oberbegriff des Anspruchs 7. Sie wird ferner gelöst durch ein digitales Speichermedium gemäß Anspruch 15. Da die hiermit erzielbaren Vorteile ungeschmälert jenen oben erläuterten entsprechen, wird zur Vermeidung von Wiederholung an dieser Stelle ausdrücklich auf ihre obige Diskussion verwiesen. Dasselbe gilt für die oben dargelegte Beschreibung des Instrumentes oder andere strukturelle Ausgestaltungen und Merkmale der erfindungsgemäßen Vorrichtung.

In bestimmten Ausführungsformen ist die erfindungsgemäße Vorrichtung ausgestaltet und vorgesehen und/oder konfiguriert, um das erfindungsgemäße Verfahren hiermit ausführen zu können. In manchen Ausführungsformen weist sie eine entsprechend konfigurierte Rechen- oder Steuereinrichtung auf. In bestimmten Ausführungsformen weist sie entsprechende, zum Ausführen der einzelnen Schritte des erfindungsgemäßen Verfahrens geeignete und/oder vorgesehene und/oder konfigurierte, Einrichtungen auf.

In einigen erfindungsgemäßen Ausführungsformen weist die Vorrichtung wenigstens einen ersten Abschnitt und wenigstens einen zweiten Abschnitt auf. Der erste Abschnitt weist wenigstens einen ersten Sensor auf, der zweite Abschnitt weist wenigstens einen zweiten Sensor auf. Zwischen dem ersten Abschnitt und dem zweiten Abschnitt ist wenigstens eine Deformationsstelle vorgesehen.

Diese Deformationsstelle ist in manchen erfindungsgemäßen Ausführungsformen als eine selbsthaltende Deformationsstelle ausgestaltet, also als eine Deformationsstelle (oder Biegestelle), welche plastisch verformbar ist oder welche nach ihrem Deformieren oder Biegen in der deformierten oder gebogenen Stellung oder Haltung verbleibt.

In gewissen erfindungsgemäßen Ausführungsformen sind der erste Abschnitt und/oder der zweite Abschnitt variabel definierbar oder festlegbar vorgesehen.

In einigen erfindungsgemäßen Ausführungsformen ist zwischen dem ersten Sensor und dem zweiten Sensor wenigstens eine Deformationsstelle (z. B. ein Gelenk oder ein Biegebereich) vorgesehen.

In gewissen Ausführungsformen weist die Deformationsstelle und/oder der Deformationsbereich eine Legierung auf.

In einigen Ausführungsformen ist die Legierung ein weichgeglühtes Material oder weist ein solches auf.

Im Folgenden werden das erfindungsgemäße Verfahren und die erfindungsgemäße Vorrichtung anhand der beigefügten Zeichnung, in welcher identische Bezugszeichen gleiche oder identische Bauteile bezeichnen, exemplarisch erläutert. In den zum Teil stark vereinfachten Figuren gilt:

Fig. 1   zeigt schematisch vereinfacht ein Instrument einer Ausführungsform mit zwei Sensoren und zwei Abschnitten im Normalzustand, geeignet zur Verwendung mit dem erfindungsgemäßen Verfahren;

Fig. 2   zeigt schematisch den Ablauf des erfindungsgemäßen Verfahrens;

Fig. 3   zeigt das Instrument der Fig. 1 in einem bestimmungsgemäß deformierten Zustand zu einem Zeitpunkt $t_2$, bei dem eine Deformation erkannt wird;

Fig. 4   zeigt das Instrument der Fig. 1 in einem bestimmungsgemäß deformierten Zustand zu einem Zeitpunkt $t_3$, bei dem der Deformationszustand als Normalzustand gesetzt wurde;

Fig. 5   zeigt das Instrument der Fig. 1 bei angedeuteter gestörter Messung der Position des ersten Sensors; und

Fig. 6   zeigt das Instrument der Fig. 1 bei angedeuteter gestörter Messung der Position des zweiten Sensors.

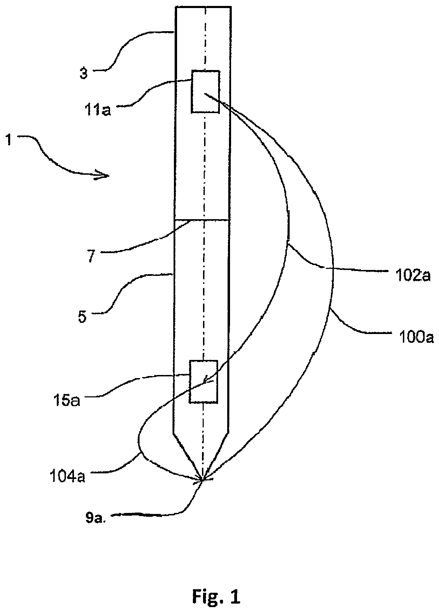

**Fig. 1** zeigt schematisch vereinfacht ein Instrument 1 mit einem ersten Abschnitt 5 und einem zweiten Abschnitt 3. Das Instrument weist zwischen den Abschnitten eine Trennfuge 7 auf, welche als Gelenk zwischen den Abschnitten 3 und 5 zur bestimmungsgemäß vorgesehenen Deformation des Instruments 1 bei dessen Gebrauch verstanden werden kann. In Fig. 1 ist das Instrument 1 nicht deformiert. Eine Abwinkelung im Bereich der Trennfuge 7 liegt nicht vor. Am Ende des ersten Abschnitts 5 befindet sich eine Instrumentenspitze 9a. Der erste Abschnitt 5 weist einen ersten Sensor 15a auf, der zweite Abschnitt 3 einen zweiten Sensor 11a.

Eine Deformation des Instruments kann auch an mehreren Trennfugen oder ohne definierte Trennfuge 7 erfolgen. Auch vollständig elastische oder plastische Verformungen sind möglich.

Die Koordinatentransformation zwischen dem ersten Sensor 15a und der Instrumentenspitze 9a wird durch eine Transformationsmatrix 104a dargestellt. Eine weitere Koordinatentransformation zwischen dem zweiten Sensor 11a und der Instrumentenspitze 9a wird durch eine Transformationsmatrix 102a dargestellt. Durch die Koordinatentransformationen 104a und 102a wird aus Sensorwerten (Lagedaten) des ersten und zweiten Sensors die Lage der Instrumentenspitze 9a berechnet. Es liegen folglich zwei Messungen für die Instrumentenspitze 9a zu einem Zeitpunkt vor. Diese sind, wie in Fig. 1 dargestellt, im ungestörten und nicht deformierten Zustand (nahezu) identisch.

Weiterhin wird eine Koordinatentransformation zwischen dem ersten Sensor 15a und einem zweiten Sensor 11a durch eine Transformationsmatrix 102a zum ersten Zeitpunkt $t_1$ dargestellt.

Der Zustand, wie er in Fig. 1 dargestellt ist, wird im Folgenden als Normalzustand angesehen.

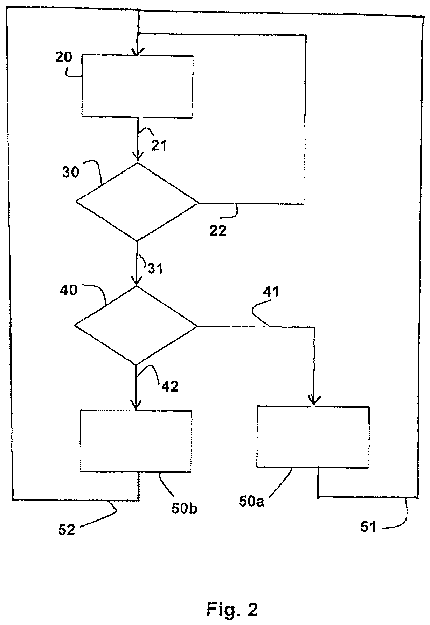

Fig. 2 zeigt ein Ablaufschema des beschriebenen Verfahrens. Zu einem ersten Zeitpunkt $t_1$ liegt in 20 das Instrument wie in Fig. 1 beschrieben im Normalzustand vor.

Zu dem nachfolgenden Zeitpunkt $t_2$ (21: Sprung zu 30) wird in 30 die Transformationsmatrix 102a zwischen dem ersten Sensor 15a und dem zweiten Sensor 11a betrachtet. Tritt keine Änderung zum Zeitpunkt $t_2$ gegenüber dem Zeitpunkt $t_1$ auf, so ist der Normalzustand weiterhin gegeben (31: Sprung zu 20). Tritt eine Änderung auf, so liegt eine Deformation oder eine Störung vor (32).

Mittels des erfindungsgemäßen Verfahrens wird in 40 geprüft, ob zum dritten Zeitpunkt $t_3$ unverändert die Gegebenheiten vorliegen, wie sie zum zweiten Zeitpunkt $t_2$ vorlagen. Diese Prüfung kann unter Einsatz eines Kriteriums erfolgen. Liegt die Gegebenheit nicht unverändert vor, wird auf eine Störung geschlossen und das Verfahren in 50a weitergeführt (41). Liegt die Gegebenheit unverändert vor, wird auf eine Deformation geschlossen und das Verfahren in 50b weitergeführt (42).

Bei Vorliegen einer Störung werden die Messwerte in 50a beispielsweise durch einen Filter bearbeitet. Nach der Beseitigung der Störung liegt wieder der Normalzustand vor (51: Sprung zu 20).

Bei Vorliegen einer Deformation wird in 50b die neue Anordnung der Sensoren zueinander (bedingt durch die Deformation) als Normalzustand gesetzt und die neue Transformation von dem zweiten Sensor 11a zur Instrumentenspitze 9a berechnet und ab diesem Zeitpunkt als Normalzustand verwendet (52: Sprung zu 20).

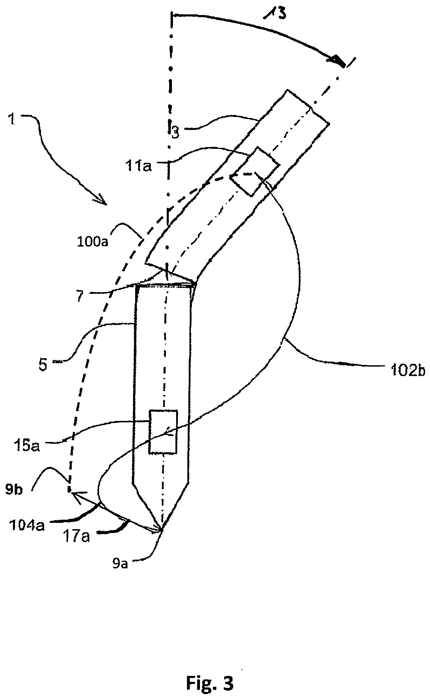

**Fig. 3** zeigt das Instrument 1 aus Fig. 1 zu einem zweiten Zeitpunkt $t_2$ in einer Stellung, in welcher der zweite Abschnitt 3 bestimmungsgemäß um einen Winkel 13 gegenüber dem ersten Abschnitt 5 verdreht oder gekippt ist.

Dadurch wird die Lage der Instrumentenspitze 9a durch Anwendung der Koordinatentransformation 100a vom zweiten Sensor 11a in seiner neuen Lage nicht korrekt dargestellt, sondern in einer um die Distanz 17a verschobenen Lage 9b. Die Koordinatentransformation 104a vom ersten Sensor 15a stellt weiterhin die Lage der Instrumentenspitze 9a korrekt dar.

Weiterhin wird eine Koordinatentransformation zwischen dem ersten Sensor 15a und dem zweiten Sensor 11a durch eine homogene Transformationsmatrix 102b zum zweiten Zeitpunkt $t_2$ dargestellt. Diese ist zu der Transformationsmatrix 102a zum ersten Zeitpunkt $t_1$ verändert.

Diese Transformationsmatrix 102b beruht offensichtlich auf korrekten Messergebnissen, was den zweiten Sensor 11a betrifft. Tatsächlich ist der zweite Abschnitt 3 zum zweiten Zeitpunkt $t_2$, welcher in Fig. 2 gezeigt ist, gegenüber seiner Lage im Raum zum ersten Zeitpunkt $t_1$ und auch gegenüber dem ersten Abschnitt 5 durch bestimmungsgemäße Benutzung des Instruments 1 deformiert.

Sowohl die zum zweiten Zeitpunkt $t_2$ veränderte Transformationsmatrix 102b (gegenüber der Transformationsmatrix 102a zum ersten Zeitpunkt $t_1$), als auch die Distanz 17a zwischen der Instrumentenspitze 9a gemessen durch den ersten Sensor 15a und dem verschobenen gemessenen Punkt 9b durch den zweiten Sensor, weisen darauf hin, dass eine Deformation oder ein Störungszustand vorliegen.

Für die Frage, ob diese festgestellte Deformation nun tatsächlich vorliegt oder Ergebnis von Messfehlern oder Fehlern einer Auswertung ist, kann mittels des erfindungsgemäßen Verfahrens geprüft werden, ob zu einem späteren dritten Zeitpunkt $t_3$ unverändert die Gegebenheiten vorliegen, wie sie zum zweiten Zeitpunkt $t_2$ vorlagen. Diese Prüfung kann unter Einsatz eines Kriteriums erfolgen.

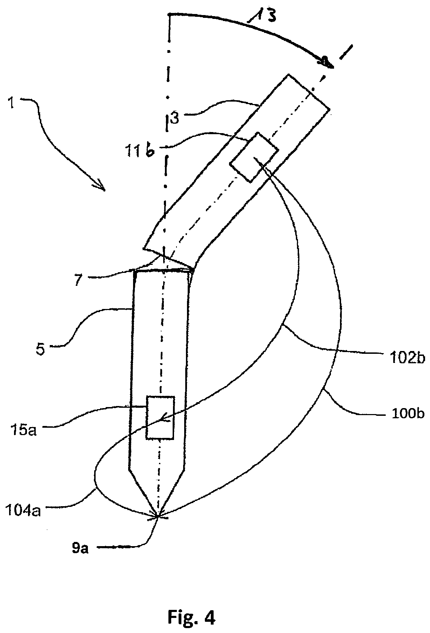

**Fig. 4** zeigt das Instrument der Fig. 3 zu einem dritten Zeitpunkt $t_3$. Mittels des erfindungsgemäßen Verfahrens wurde geprüft, ob die Gegebenheit, wie sie zum zweiten Zeitpunkt $t_2$, vorlag, weiterhin unverändert vorliegt. Dadurch kann auf das tatsächliche Vorliegen einer Deformation geschlossen werden.

Der Deformationszustand wird von diesem Zeitpunkt als neuer Normalzustand angesehen. Dafür wird der Unterschied zwischen der Transformation 102a zum ersten Zeitpunkt und der Transformation 102b zum zweiten bzw. dritten Zeitpunkt berechnet. Dieser wird dann der Transformation 100a vom zweiten Sensor 11a zur Instrumentenspitze 9a hinzugefügt, so dass die daraus resultierende neue Transformation 100b, die Lage der Instrumentenspitze 9a aus Sensorwerten des zweiten Sensors 11a wieder korrekt berechnet.

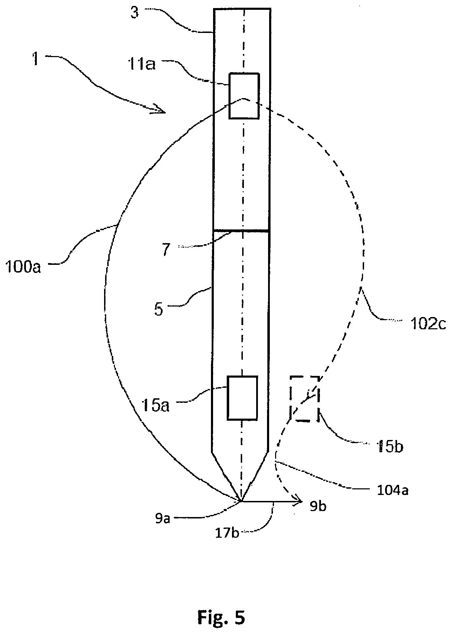

**Fig. 5** zeigt das Instrument der Fig. 1. Die Koordinatentransformation zwischen dem zweiten Sensor 11a und der Instrumentenspitze 9a wird durch die Transformationsmatrix 100a dargestellt.

Hervorgerufen durch eine Störung bei der Messung des ersten Sensors 15a wird allerdings eine verschobene Position 15b des ersten Sensors gemessen. Dadurch wird auch durch Verwendung der Koordinatentransformation 104a die Position der Instrumentenspitze 9a um eine Distanz 17b verschoben gemessen (der Abschnitt 1 wird als starr angesehen).

Dadurch wird die Koordinatentransformation zwischen dem zweiten Sensor 11a und der neuen, fehlerbehaftet gemessenen Position 15b des ersten Sensors durch die homogene Transformationsmatrix 102c dargestellt.

Die Koordinatentransformation 100a zwischen dem zweiten Sensor 11a und der Instrumentenspitze 9a bleibt unverändert. Dabei wird hier angenommen, dass die Störung der Messung des ersten Sensors 15a mit dem Ergebnis der neuen, gemessenen Position 15b keine Auswirkung auf die Messung des Sensors 11a hat.

Der fehlerhafte Charakter der ermittelten Position 15b des ersten Sensors kann durch Kontrolle der Position des ersten Sensors des ersten Abschnitts 3 zu einem dritten Zeitpunkt $t_3$ erfolgen, wie oben beschrieben.

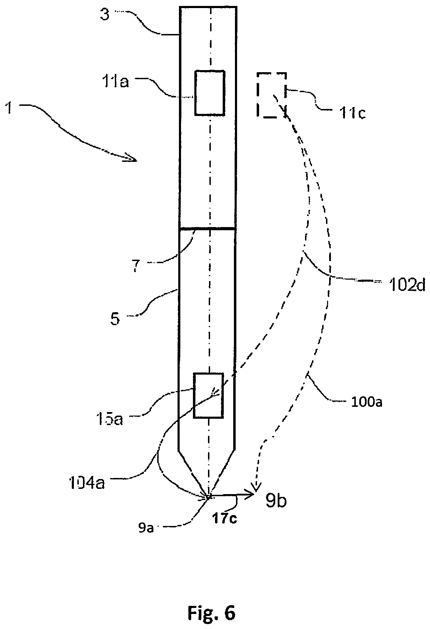

**Fig. 6** zeigt vereinfacht das Instrument 1 aus Fig. 1 in seinem nicht deformierten Zustand der Fig. 3.

Gegenüber Fig. 5 ist jetzt die Messung des zweiten Sensors 11a gestört. Diese wird daher als fehlerhafte Position 11c gemessen.

Dadurch wird durch die Koordinatentransformation 100a von dem zweiten Sensor 11a, jetzt gemessen als Position 11c, nicht mehr die Instrumentenspitze 9a gemessen, sondern eine um eine Distanz 17c verschobene Instrumentenspitze 9b.

Auch wird die Koordinatentransformation zwischen dem zweiten Sensor, gemessen als Position 11c, und dem ersten Sensor 15a durch die neue homogene Transformationsmatrix 102d dargestellt.

Die Koordinatentransformation 104a zwischen dem ersten Sensor 15a und der Instrumentenspitze 9 bleibt unverändert. Dabei wird angenommen, dass die Störung der Messung des Sensors 11a (mit dem Ergebnis der fehlerhaft angenommenen Position 11c) keine Auswirkung auf die Messung des Sensors 15a hat.

Wie zu den Fig. 3 bis 5 beschrieben, lässt sich auch beim Instrument der Fig. 6 durch eine Kontrolle, ob die veränderte Position des ersten Sensors oder des zweiten Sensors auch noch zu einem dritten Zeitpunkt $t_3$ vorliegt, feststellen, ob die gemessene Positionsänderung ein Artefakt darstellt oder einer tatsächlichen Positionsänderung entspricht.

**Patentansprüche**

1. Verfahren zur Überprüfung von Lage- oder Positionsdaten eines medizinischen Instrumentes (1) mit wenigstens einem ersten Abschnitt (5) und wenigstens einem zweiten Abschnitt (3), wobei der erste Abschnitt (5) wenigstens einen ersten Sensor (15a) aufweist und der zweite Abschnitt (3) wenigstens einen zweiten Sensor (11a) aufweist, und wobei das Verfahren ein messtechnisches Bestimmen der Lage oder Position, oder deren Veränderung, des ersten Sensors (15a) und des zweiten Sensors (11a) umfasst;
**gekennzeichnet durch** die Schritte

     - Bestimmen einer Ausprägung eines veränderlichen Merkmals des räumlichen Bezugs zwischen der Lage oder Position des ersten Sensors (15a) und/oder wenigstens eines Punktes auf dem ersten Abschnitt (5) einerseits und der Lage oder Position des zweiten Sensors (11a) und/oder wenigstens eines Punktes auf dem zweiten Abschnitt (3) oder dem ersten Abschnitt (5), der durch den zweiten Sensor referenziert wird, andererseits, zu jeweils wenigstens einem ersten Zeitpunkt ($t_1$), zu einem zweiten Zeitpunkt ($t_2$) und zu einem dritten Zeitpunkt ($t_3$) ;
     - Ermitteln anhand eines Kriteriums, ob ein Unterschied in der Ausprägung des veränderlichen Merkmals zwischen einer ersten Ausprägung zum ersten Zeitpunkt ($t_1$) und einer zweiten Ausprägung zum zweiten Zeitpunkt ($t_2$) zum dritten Zeitpunkt ($t_3$) noch besteht,

sodass festgestellt werden kann, ob eine Störung vorliegt.

2. Verfahren nach Anspruch 1, wobei basierend auf der Ermittlung anhand des Kriteriums eine Unterscheidung getroffen wird, ob der Unterschied in der Ausprägung des veränderlichen Merkmals zwischen der ersten Ausprägung zum ersten Zeitpunkt ($t_1$) und der zweiten Ausprägung zum zweiten Zeitpunkt ($t_2$) auf einer Deformation des Instruments (1) oder auf einer anderen Beeinflussung des gemessenen räumlichen Bezugs beruht.

3. Verfahren nach Anspruch 1 oder 2, wobei wenigstens ein weiterer Sensor auf jeweils einem weiteren Abschnitt verwendet wird, wobei der räumliche Bezug zwischen Lage- oder Position des oder jedes einzelnen weiteren Sensors einerseits und dem ersten Sensor (15a) und/oder wenigstens eines Punktes auf dem ersten Abschnitt (5) anderseits bestimmt wird.

4. Verfahren nach einem der vorangegangenen Ansprüche, wobei die Änderung des räumlichen Bezugs des oder der Sensoren und/oder Störeinflüsse bei dem Bestimmen von Positionsdaten unter Anwendung des Kriteriums unter Beachtung eines vorbestimmten Toleranzbereichs und/oder eines vorbestimmten Grenzwerts bestimmt wird.

5. Verfahren nach einem der vorangegangenen Ansprüche, wobei bei einer bleibenden Änderung des räumlichen Bezugs des oder der Sensoren die Ausprägung des veränderlichen Merkmals des räumlichen Bezugs zu einem dritten Zeitpunkt ($t_3$) als neue Ausgangsausprägung des Verfahrens gesetzt wird.

6. Verfahren nach einem der vorangegangenen Ansprüche, wobei eine Spitze des Instruments (1) der Punkt auf dem ersten Abschnitt (5) des Instrumentes (1) ist.

7. Vorrichtung, welche ein medizinisches Instrument ist oder aufweist, welche wenigstens einen ersten Abschnitt (5) und wenigstens einen zweiten Abschnitt (3) aufweist, wobei der erste Abschnitt (5) wenigstens einen ersten Sensor (15a) aufweist, wobei der zweite Abschnitt (3) wenigstens einen zweiten Sensor (11a) aufweist,
wobei die Vorrichtung Mittel zum Bestimmen einer Ausprägung eines veränderlichen Merkmals des räumlichen Bezugs zwischen der Lage oder Position des ersten Sensors (15a) und/oder wenigstens eines Punktes auf dem ersten Abschnitt (5) einerseits und der Lage oder Position des zweiten Sensors (11a) und/oder wenigstens eines Punktes auf dem zweiten Abschnitt (3) oder dem ersten Abschnitt (5), der durch den zweiten Sensor referenziert wird, andererseits, zu jeweils wenigstens einem ersten Zeitpunkt (t1), zu einem zweiten Zeitpunkt (t2) und zu einem dritten Zeitpunkt (t3) umfasst,

- wobei die Vorrichtung zudem Mittel zum Ermitteln anhand eines Kriteriums, ob ein Unterschied in der Ausprägung des veränderlichen Merkmals zwischen einer ersten Ausprägung zum ersten Zeitpunkt (t1) und einer zweiten Ausprägung zum zweiten Zeitpunkt (t2) zum dritten Zeitpunkt (t3) noch besteht, umfasst,

sodass festgestellt werden kann, ob eine Störung vorliegt.

8. Vorrichtung nach Anspruch 7, wobei zwischen dem ersten Abschnitt (5) und dem zweiten Abschnitt (3) eine Deformationsstelle, insbesondere eine selbsthaltende Deformationsstelle, vorgesehen ist.

9. Vorrichtung nach Anspruch 7 oder 8, wobei wenigstens einer der Sensoren (11a, 15a) eine oder mehrere Spulen beinhaltet oder hieraus besteht.

10. Vorrichtung nach Anspruch 9, wobei die Spule wenigstens ein durchgängiges Inneres aufweist.

11. Vorrichtung nach Anspruch 9 oder 10, wobei die Spule um einen Abschnitt des Instruments oder innerhalb des Abschnitts gewickelt ist.

12. Vorrichtung nach Anspruch 11, wobei der Abschnitt des Instruments einen rohrförmigen (hohlen) Querschnitt hat.

13. Vorrichtung nach Anspruch 11 oder 12, wobei der Abschnitt des Instruments einen nicht hohlen Querschnitt hat.

14. Vorrichtung nach einem der Ansprüche 8 bis 13, wobei zwischen einem ersten Sensor (15a) und einem zweiten Sensor (11a) eine Deformationsstelle vorgesehen ist.

15. Digitales Speichermedium, insbesondere Diskette, CD oder DVD, mit elektrisch auslesbaren Steuersignalen, die derart mit einem programmierbaren Computersystem zusammenwirken können, dass die Schritte eines Verfahrens gemäß einem der Ansprüche 1 bis 6 veranlasst werden.

**Claims**

1. A method for checking location or position data of a medical instrument (1) having at least one first portion (5) and at least one second portion (3), wherein the first portion (5) comprises at least one first sensor (15a) and the second portion (3) comprises at least one second sensor (11a), and wherein the method includes a metrological determination of the location or position, or the modification thereof, of the first sensor (15a) and of the second sensor (11a); **characterized by** the following steps:

- determining an expression of a variable feature of the spatial reference between the location or position of the first sensor (15a) and/or at least of one point on the first portion (5) on the one hand, and the location or position of the second sensor (11a) and/or at least of one point on the second portion (3) or on the first portion (5), which is referenced by the second sensor, on the other hand, at least at a first moment ($t_1$), at a second moment ($t_2$), and at a third moment ($t_3$) respectively;
- identifying, according to a criterion, whether a difference in the expression of the variable feature between a first expression at the first moment ($t_1$) and a second expression at the second moment ($t_2$), is still present at the third moment ($t_3$),

so that it can be determined whether a perturbation exists.

2. The method according to claim 1, wherein based on the identification by means of the criterion, it is distinguished whether the difference in the expression of the variable feature between the first expression at the first moment ($t_1$) and the second expression at the second moment ($t_2$) is due to a deformation of the instrument (1) or to another influence of the measured spatial reference.

3. The method according to claim 1 or 2, wherein at least one further sensor is used on respectively one further portion, the spatial reference being determined between the location or position of the or of each individual further sensor on the one hand, and the first sensor (15a) and/or at least one point on the first portion (5) on the other hand.

4. The method according to anyone of the preceding claims, wherein the change in the spatial reference of the sensor (s) and/or perturbation influences in the determination of position data is determined using the criterion and taking into consideration a predetermined tolerance range and/or a predetermined limit value.

5. The method according to anyone of the preceding claims, wherein in the event of a permanent change in the spatial reference of the sensor (s), the expression of the variable feature of the spatial reference at a third moment ($t_3$) is set as a new output expression of the method.

6. The method according to anyone of the preceding claims, wherein a tip of the instrument (1) is the point on the first portion (5) of the instrument (1).

7. An apparatus, being or comprising a medical instrument, which comprises at least a first portion (5) and at least a second portion (3), the first portion (5) comprising at least one first sensor (15a), the second portion (3) comprising at least one second sensor (11a),

   - wherein the apparatus includes means for determining an expression of a variable feature of the spatial reference between the location or position of the first sensor (15a) and/or at least of one point on the first portion (5) on the one hand, and the location or position of the second sensor (11a) and/or at least of one point on the second portion (3) or on the first portion (5), which is referenced by the second sensor, on the other hand, at least at a first moment ($t_1$), at a second moment ($t_2$), and at a third moment ($t_3$) respectively;
   - wherein the apparatus further includes means for identifying, according to a criterion, whether a difference in the expression of the variable feature between a first expression at a first moment ($t_1$) and a second expression at a second moment ($t_2$), is still present at the third moment ($t_3$);

   so that it can be determined whether a perturbation exists.

8. The apparatus according to claim 7, wherein a deformation site, in particular a self-holding deformation site, is provided between the first portion (5) and the second portion (3).

9. The apparatus according to claim 7 or 8, wherein at least one of the sensors (11a, 15a) includes or consists of one or more coils.

10. The apparatus according to claim 9, wherein the coil comprises at least one continuous interior.

11. The apparatus according to claim 9 or 10, wherein the coil is wound around a portion of the instrument or within the portion.

12. The apparatus according to claim 11, wherein the portion of the instrument has a tubular (hollow) cross-section.

13. The apparatus according to claim 11 or 12, wherein the portion of the instrument has a non-hollow cross-section.

14. The apparatus according to anyone of the claims 8 to 13, wherein a deformation site is provided between a first sensor (15a) and a second sensor (11a).

15. A digital storage medium, in particular a diskette, a CD or a DVD, comprising electrically readable control signals, which can interact with a programmable computer system in such a way that the steps of a method according to anyone of claims 1 to 6 are triggered.

**Revendications**

1. Un procédé de vérification de données d'emplacement ou de positionnement d'un instrument médical (1) ayant au moins une première section (5) et au moins une seconde section (3), la première section (5) comprenant au moins un premier capteur (15a) et la seconde section (3) comprenant au moins un second capteur (11a), le procédé incluant une détermination métrologique de l'emplacement ou du positionnement du premier capteur (15a) et du second capteur (11a), ou de la modification de celui-ci ; **caractérisé par** les étapes suivantes:

   - déterminer une expression d'une caractéristique variable de la référence spatiale entre l'emplacement ou le positionnement du premier capteur (15a) et/ou d'au moins un point sur la première section (5) d'une part, et l'emplacement ou le positionnement du second capteur (11a) et/ou d'au moins un point sur la seconde section (3) ou sur la première section (5), qui est référencée par le second capteur, d'autre part, au moins à un premier instant ($t_1$), à un second instant ($t_2$), et à un troisième instant ($t_3$) respectivement;
   - identifier, sur la base d'un critère, si une différence dans l'expression de la caractéristique variable entre une première expression au premier instant ($t_1$) et une seconde expression au second instant ($t_2$), existe encore au troisième instant ($t_3$),

   afin de pouvoir déterminer s'il y a une perturbation.

2. Le procédé selon la première revendication, où, sur la base de l'identification au moyen du critère, on distingue si la différence dans l'expression de la caractéristique variable entre la première expression au premier instant ($t_1$) et la seconde expression au

second instant ($t_2$) est due à une déformation de l'instrument (1) ou à une autre influence de la référence spatiale mesurée.

3. Le procédé selon la revendication 1 ou 2, où au moins un autre capteur est utilisé respectivement sur une autre section, la référence spatiale étant déterminée entre l'emplacement ou le positionnement du ou de chaque autre capteur individuel d'une part, et du premier capteur (15a) et/ou d'au moins un point sur la première section (5) d'autre part.

4. Le procédé selon l'une quelconque des revendications précédentes, où la modification de la référence spatiale du ou des capteur (s) et/ou des influences perturbantes dans la détermination des données de positionnement est déterminée en utilisant le critère tout en prenant en considération une plage de tolérance prédéterminée et/ou une valeur limite prédéterminée.

5. Le procédé selon l'une quelconque des revendications précédentes, où en cas de modification permanente de la référence spatiale du ou des capteur (s), l'expression de la caractéristique variable de la référence spatiale à un troisième instant ($t_3$)est fixée comme nouvelle expression de sortie du procédé.

6. Le procédé selon l'une quelconque des revendications précédentes, où une pointe de l'instrument (1) est le point sur la première section (5) de l'instrument (1).

7. Un appareil, étant ou comprenant un instrument médical, comprenant au moins une première section (5) et au moins une seconde section (3), la première section (5) comprenant au moins un premier capteur (15a), la seconde section (3) comprenant au moins un second capteur (11a),

   - où l'appareil inclut des moyens permettant de déterminer une expression d'une caractéristique variable de la référence spatiale entre l'emplacement ou le positionnement du premier capteur (15a) et/ou d'au moins un point sur la première section (5) d'une part, et l'emplacement ou le positionnement du second capteur (11a) et/ou d'au moins un point sur la seconde section (3) ou sur la première section (5), qui est référencée par le second capteur, d'autre part, au moins à un premier instant ($t_1$), à un second instant ($t_2$), et à un troisième instant ($t_3$) respectivement;
   - où l'appareil inclut en outre des moyens permettant d'identifier, sur la base d'un critère, si une différence dans l'expression de la caractéristique variable entre une première expression au premier instant ($t_1$) et une seconde expression au second instant ($t_2$), existe encore au troisième instant ($t_3$),

afin de pouvoir déterminer s'il y a une perturbation.

8. L'appareil selon la revendication 7, où un site de déformation, notamment un site de déformation autotenant, est prévu entre la première section (5) et la seconde section (3).

9. L'appareil selon la revendication 7 ou 8, où au moins l'un des capteurs (11a, 15a) comprend ou consiste en une ou plusieurs bobines.

10. L'appareil selon la revendication 9, où la bobine comprend au moins un intérieur continu.

11. L'appareil selon la revendication 9 ou 10, où la bobine est enroulée autour d'une section de l'instrument ou à l'intérieur de la section.

12. L'appareil selon la revendication 11, où la section de l'instrument a une section transversale tubulaire (creuse).

13. L'appareil selon la revendication 11 ou 12, où la section de l'instrument a une section transversale noncreuse.

14. L'appareil selon l'une quelconque des revendications 8 à 13, où un site de déformation est prévu entre un premier capteur (15a) et un second capteur (11a).

15. Un support de stockage numérique, notamment une disquette, un cd ou un dvd comportant des signaux de commandes lisibles électriquement, qui peuvent interagir avec un système informatique programmable de telle sorte que les étapes d'un procédé selon l'une quelconque des revendications 1 à 6 soient déclenchées.

**Fig. 1**

Fig. 2

**Fig. 3**

**Fig. 4**

**Fig. 5**

**Fig. 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009021705 A1
- EP 1632168 A1